# EUROPEAN PATENT SPECIFICATION

(11) **EP 4 203 712 B1**
(45) Date of publication and mention of the grant of the patent: **05.11.2025**
(21) Application number: 21766482.0
(22) Date of filing: 27.08.2021
(51) Int. Cl.: A23L 33/21, A23L 33/00, G16H 20/60

(54) **PERSONALISATION OF DIETARY FIBER COMPOSITIONS, METHODS AND SYSTEMS THEREOF**
PERSONALISIERUNG VON BALLASTSTOFFZUSAMMENSETZUNGEN, VERFAHREN UND SYSTEME DAFÜR
PERSONNALISATION DE COMPOSITIONS EN FIBRES ALIMENTAIRES, PROCÉDÉS ET SYSTÈMES ASSOCIÉS

(30) Priority: 31.08.2020 EP 20193534; 22.12.2020 US 202063129159 P
(43) Date of publication of application: 05.07.2023
(73) Proprietor: Société des Produits Nestlé S.A., 1800 Vevey (CH)
(72) Inventor: BERGER, Bernard, 1613 MARACON (CH); EMAMI, Nashmil, 1110 Morges (CH); LAMOTHE, Lisa Marcela, 1000 Lausanne 26 (CH); LE ROY, Caroline, 1007 Lausanne (CH)
(74) Representative: Kiwit, Benedikt
(86) International application number: PCT/EP2021/073698
(87) International publication number: WO 2022/043475

(56) References cited:
- WO-A1-2019/093957
- US-A1- 2010 113 892
- US-A1- 2013 261 183
- US-A1- 2018 144 820
- US-A1- 2019 145 988
- US-A1- 2019 374 570
- US-A1- 2020 051 693
- GARRON MARIE-LINE ET AL: "The continuing expansion of CAZymes and their families", CURRENT OPINION IN CHEMICAL BIOLOGY, CURRENT BIOLOGY LTD, LONDON, GB, vol. 53, 21 September 2019 (2019-09-21), pages 82 - 87, XP085968286, ISSN: 1367-5931, [retrieved on 20190921], DOI: 10.1016/J.CBPA.2019.08.004
- KAMALDEEP KAUR ET AL: "Metagenomics analysis reveals features unique to Indian distal gut microbiota", PLOS ONE, vol. 15, no. 4, 8 April 2020 (2020-04-08), pages e0231197, XP055771839, DOI: 10.1371/journal.pone.0231197
- TANUDEEP BHATTACHARYA ET AL: "Global Profiling of Carbohydrate Active Enzymes in Human Gut Microbiome", PLOS ONE, vol. 10, no. 11, 6 November 2015 (2015-11-06), pages e0142038, XP055771863, DOI: 10.1371/journal.pone.0142038
- DAVIDE A. CECCHINI ET AL: "Functional Metagenomics Reveals Novel Pathways of Prebiotic Breakdown by Human Gut Bacteria", PLOS ONE, vol. 8, no. 9, 16 September 2013 (2013-09-16), pages e72766, XP055291405, DOI: 10.1371/journal.pone.0072766

## Description

### FIELD OF THE INVENTION

The present invention provides methods to personalise dietary fiber for an individual based on their CAZyme profile and recommendations for dietary fiber products and compositions as well as systems to facilitate quick and easy use.

### BACKGROUND TO THE INVENTION

Document WO 2019/093957 A1 discloses a method for controlling fiber intake in mammals, comprising the steps: a) providing at least one input data; b) based on said input data, generating at least one output data; wherein the input data is based on information selected from the group consisting of host DNA, host metabolism, host colonic microbial composition, gene activity, metabolic activity, host specific parameters, and any combinations thereof wherein the output data is selected from the group consisting of information on fiber consumption, fiber supplementation and, any combinations thereof. Document
US 2020/051693 A1 discloses systems and methods for developing individualized dietary and health improvement plans based on an individual's intestinal microbiome and digestive activity. A system provides a hydrogen and/or methane sensor device and a wireless platform in communication with the sensor device to periodically analyze the individual's metabolic activity in correlation with their gut microbiome and a personal database to provide personalized feedback to the individual of treatment plans and general techniques that can be used to improve the individual's general health and well being. Document
US 2018/144820 A1 discloses a system for recommending foods to a user based on health data, the system including a database, a memory and a processor. The database stores user health data for each user within a plurality of users, including vitals, genotypic and phenotypic data, user food preference data and foods data that includes macronutrient and micronutrient data for foods that may be recommended to a user. The memory stores program instructions, including program instructions that are capable of (i) classifying user health data into predetermined diet types and micronutrient recommendations, (ii) filtering the food data to determine available foods for a user; (iii) a ranking available meals for the user based on the micronutrient recommendations and the food data, and (iv) translating micronutrient recommendations and/or food data for the available foods for the user into specific food recommendations for the user.
Document US 2010/113892 A1 discloses a method for determining personalized nutrition and diet using nutrigenomics and physiological data.

Dietary fiber is an essential part of any healthy diet. Within the diet, fiber is mostly found in vegetables, fruits, whole grains, and legumes. Fiber is generally classified in two types: soluble and insoluble, which both play important roles in health. Insoluble fiber does not dissolve in water and adds bulk to the stool, preventing constipation. Soluble fiber absorbs water, forming a gel-like substance in the digestive system. Soluble fiber may help lower cholesterol levels and help regulate blood sugar levels which is crucial in reducing the risk of chronic health conditions [Threapleton et al., 2013].

Despite the known association of dietary fiber and health outcomes, most people do not get enough fiber from their diets. According to some estimates, only 5% of the population meet the adequate dietary fiber intake recommendations in Western countries such as the United States. This means that most people could benefit from increasing their daily fiber intake.

The beneficial effects of fibers on health are principally mediated by gut microbes that have the capability to degrade these complex fiber structures to produce metabolites such as short chain fatty acids (SCFAs) [Makki et al., 2018]. The capability of microbes to degrade dietary fibers depends on the genes encoding for carbohydrate-active enzymes (CAZymes). Accordingly, the composition of the microbiome will affect the individual's ability to utilize fibers, and impact the fiber-associated health benefits of the host.

Carbohydrate-active enzymes (CAZymes) confer to gut microbes the capability to degrade non-digested dietary fibers. For each individual, the microbial genome encodes for a unique number and combination of CAZymes ranging from 0 to over 300 encoding genes [Flint et la., 2012], which means that an individual microbiome will differ in its capability to degrade dietary fibers.

Despite this high inter-individual variability, Bhattacharya et al., (2015) and Kaur et al., (2020) demonstrated that CAZyme profiles clusters could be identified and these correspond to different geographic regions and different food intake. They also found that the abundance of CAZymes in the human gut was negatively associated with age but positively with healthy BMI.

Habitual diet can condition the proficiency of the microbiome in exploiting certain types and quantities of dietary fiber. CAZyme profiles can be used to predict which fiber is likely to be degraded an individual's microbiome to generate products subsequently exerting positive effects on the host (Makki et al., 2018).

Clinical trials evaluating the effects of fiber supplementation on the gut microbiome and host health often report inconclusive results with part of the population responding to the fiber supplementation while others exhibit no measurable effects [Kovatcheva-Datchary., et al 2015]. This is due, in part, to microbiome diversity and the inter-individual differences in ability to utilise fibers. Simply by increasing the dietary fiber intake is no guarantee of beneficial health outcomes without considering the individual's ability to utilize the fiber based on their CAZyme profile.

To date, dietary interventions and product development have focused on increasing total fiber consumption mainly by adding soluble, prebiotic fibers, for example: inulin, FOS, GOS, without accounting for fiber diversity and considering the individual microbiome's capability to utilise those fibers. Digital methods of monitoring dietary fiber consumption, such as MyBioma or Carbiotix, also have not considered the individual microbiome's capability to utilise diverse fibers. Hence, fiber consumption may be increased without noticeable health benefits on the host individual since their microbiome may not have the set of enzymes (CAZymes) enabling the degradation of the fiber.

A further additional problem when increasing dietary fiber, particularly in amounts over 70 g per day, is that there is an undesirable side-effect in term of gut comfort which may lead to a number of different symptoms such as abdominal pain, bloating, flatulence, constipation and/or diarrhea which are detrimental to maintaining compliance of an individual to high fiber diet over the long term.

Therefore, there is a need to provide targeted dietary fiber recommendations that are personalized which can optimize the health benefits and minimize the undesirable side-effects related to gut comfort ensuring long term compliance by individuals. In addition, there is the need to have these dietary fiber recommendations delivered in a user-friendly system.

### SUMMARY OF THE INVENTION

The present invention provides methods and systems to personalize dietary fiber for an individual based on their CAZyme profile and recommendations for dietary fiber foodstuffs, food, beverage and dietary products, food and beverage compositions, and dietary supplements. Especially, the present invention provides a computer-implemented method according to independent claim 1 and a system according to independent claim 21 for providing a personalized fiber recommendation to an individual.

The method for a personalized fiber recommendation for an individual comprises determining the CAZyme profile of said individual.

The CAZyme profile of said individual is determined by a dietary assessment tool, preferably in the form of a questionnaire, such as a food frequency questionnaire.

The dietary assessment tool, preferably the food frequency questionnaire may comprise measuring the consumption of: fruits, vegetables, legumes, whole grains, nuts and seeds, potatoes, dairy products, beverages and snacks to estimate the diversity of fiber consumption within a specific period of time and to determine the CAZyme profile of said individual.

In one embodiment, said dietary assessment tool, preferably said food frequency questionnaire comprises measuring the consumption of: whole wheat flour, beans, potatoes, tomatoes, bell peppers, cauliflower, apple, banana, tea, sugar and milk to estimate the diversity of fiber consumption within a specific period of time and to determine the CAZyme profile of said individual.

In another embodiment, the CAZyme profile of said individual may be additionally or alternatively determined by a biological sample from said individual, preferably a fecal sample.

In several embodiments, the CAZyme profile of an individual is compared to reference CAZyme profiles, to determine a CAZyme Cluster reference for that individual which helps to select the personalized fiber recommendation.

In several embodiments of the invention, the personalized fiber recommendation is a daily fiber intake less than 70 g per day and determined by the gender of the individual.

In one embodiment for females, the daily fiber recommendation is preferably between 20 g to 28 g per day and not exceeding 70 g per day.

In one embodiment for males, the daily fiber recommendation is preferably between 30 g to 38 g per day and not exceeding 70 g per day.

In several embodiments, the personalized fiber recommendation is preferably fiber in the form of consumable dietary fiber.

In several embodiments, the personalized fiber recommendation is a food product, beverage product, or dietary supplement containing a high fiber foodstuff.

In several embodiments, the personalized fiber recommendation in the form of consumable dietary fiber is selected from high fiber foodstuffs comprising: fruits, vegetables, legumes, whole grains, nuts and seeds, potatoes with skin and dark chocolate in an amount not exceeding 70g of fiber per day and personalized for females in the amount between 20 g to 28 g fiber per day and for males in the amount between 30 g to 38 g fiber per day.

In some embodiments, the high fiber foodstuffs comprising fruits containing fiber in the amount of at least about 2.0 g to 10.0 g per 100g are selected from the group comprising: passionfruit 10.0 g/100g; avocado 6.7 g/100g; raspberries 6.5 g/100g; blackberries 5.3g/100g; guava 5.0 g/100g; persimmon 4.5 g/100g; mango 3.5 g/100g; pears 3.1 g/100g; banana 2.6 g/100g; apples 2.4 g/100g; blueberries 2.4 g/100g; or strawberries 2.0 g/100g.

In some embodiments, the high fiber foodstuffs comprising vegetables containing fiber in the amount of at least about 1.0 g to 10.0 g per 100 g are selected from the group comprising: artichokes 8.6 g/100g; kale 3.6 g/100g; carrots 2.8 g/100g; beets 2.8 g/100g; broccoli 2.6 g/100g; Brussel sprouts 2.6 g/100g; spinach 2.2 g/100g; cauliflower 2.0 g/100g; bell peppers 1.2 g/100g or tomatoes 1.2 g/100g.

In some embodiments, the high fiber foodstuffs comprising legumes containing fiber in the amount of at least about 5.0 g to 9.0 g per 100g are selected from the group comprising: black beans 8.7 g/100g; split peas 8.3 g/100g; lentils 7.9 g/100g; chickpeas 7.6 g/100g; kidney beans 6.4 g/100g; baked beans 5.5 g/100g; lima beans 5.3 g/100g; or edamame beans 5.2 g/100g.

In some embodiments, the high fiber foodstuffs comprising whole grains containing fiber in the amount of at least about 2.8 g to 14.5 g per 100g are selected from the group comprising: barley 17.0g/100g; whole grain flour 11 g/100g; oats 10.6 g/100g; quinoa 2.8 g/100g; or popcorn 14.5 g/100g.

In some embodiments, the high fiber foodstuffs comprising nuts and seeds containing fiber in the amount of at least about 7.0 g to 35.5 g per 100g are selected from the group comprising: chia seeds 34.4g/100g; pumpkin seeds 18.4 g/100g; almonds 12.5 g/100g; pistachios 10.0 g/100g; coconut 9.0 g/100g; sunflower seeds 8.6 g/100g; or walnuts 7.0 g/100g.

In some embodiments, the high fiber foodstuffs comprising potatoes with skin containing fiber in the amount of at least about 2.5g per 100g.

In some embodiments, the high fiber foodstuffs comprising dark chocolate containing at least 70% cocoa containing fiber in the amount of at least about 10.9 g per 100g.

In other embodiments, the personalized fiber recommendation is in the form of consumable dietary fiber and a dietary supplement.

In embodiments where a dietary fiber supplement is used, the fibre supplement is selected from a supplement containing guar fiber, psyllium, glucomannan or β-glucans.

In a further embodiment, the computer-implemented method further comprises:
(iii) delivering the personalized fiber composition.

The delivery of the personalized fiber composition may be in the form of a food product, beverage product, dietary supplement or a combination of any of these together.

In a further embodiment, the system of the invention may further comprise:
(iv) an output module for delivering the personalized dietary fiber composition.

The delivery of the personalized dietary fiber composition may be in the form of: food products, beverage products, or dietary supplements or a combination thereof in a kit of parts.

### DESCRIPTION OF FIGURES

**Figure 1****: Clustering based on CAZyme profiles**
   PCA score plot by cluster indicating 3 main clusters. Cluster 1 white circles, Cluster 2 grey circles, Cluster 3 black circles.
**Figure 2****: Contribution by weight of each CAZyme**
   This figure shows the three distinct CAZyme profiles: Cluster 1, Cluster 2 and Cluster 3 CAZyme profiles on the y-axis. The x-axis shows which CAZymes correspond to each of the three clusters respectively. The asterix (*) indicates the relevance of a particular CAZyme for each of the corresponding clusters. Positive values correspond to the enrichment of a specific CAZyme in a cluster while negative ones represent a depletion.

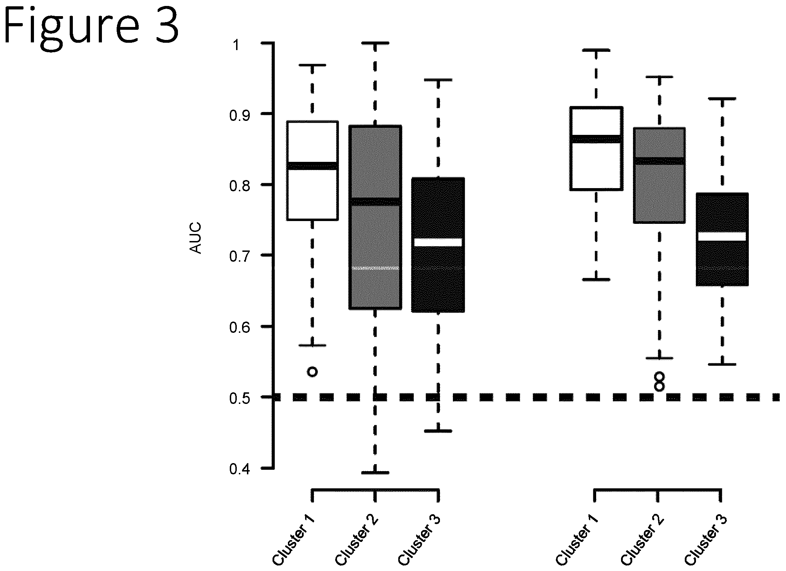

**Figure 3****: Accuracy of Predictive Model CAZyme clusters**
   Boxplot of the area under the curve (AUC) that represent the accuracy of prediction (1 would mean that 100% of predictions of clusters are correct, 0.5 would be 50% - the minimum obtained by chance) of the sample in each cluster based on food frequency questionnaire before and after model optimization (conducted by selection of the most important food variables).

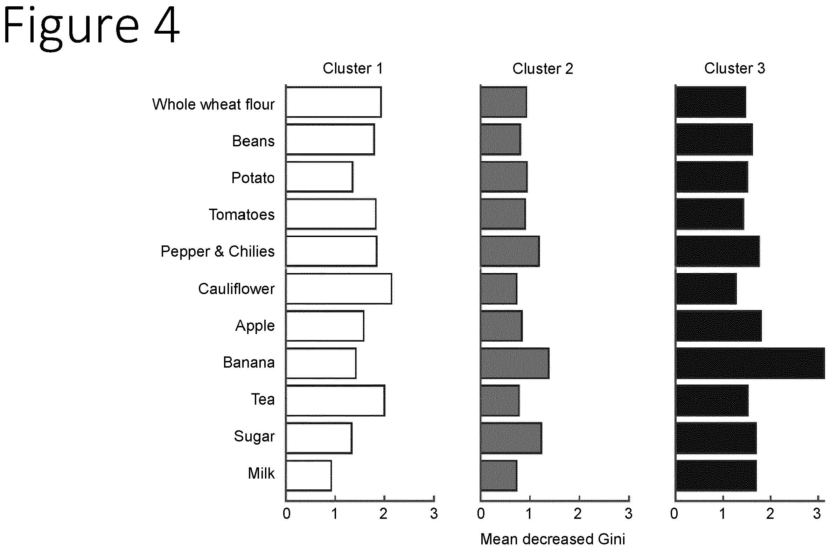

**Figure 4****: Gini coefficient per Cluster and Foodstuff category**
   The Gini coefficient is a measure of the distribution importance of contribution of foodstuff to the prediction of each cluster across the population.

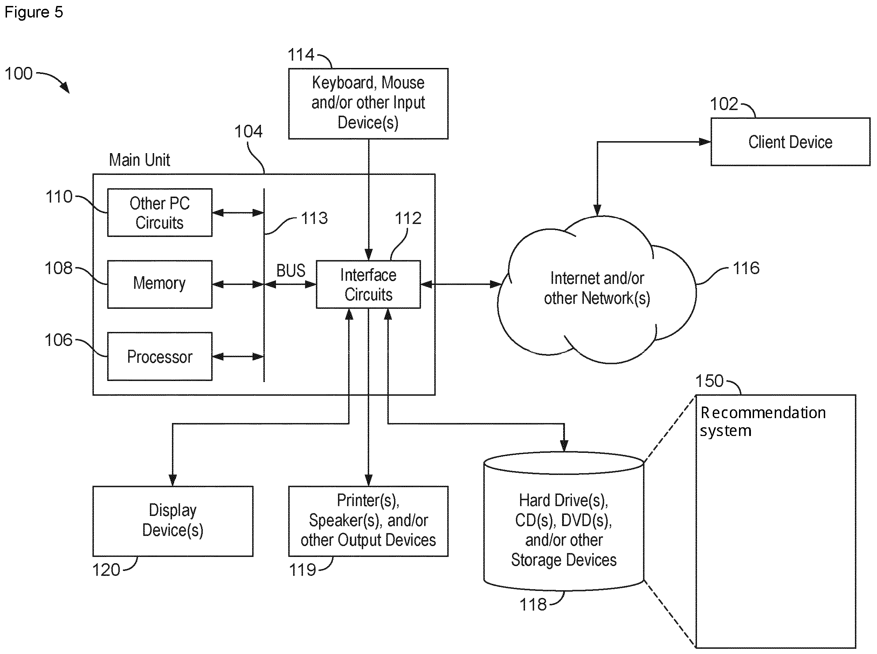

**Figure 5****: Computer implemented system**
   This figure shows the computer implemented system which is an example of a system used to determine the CAZyme profile of the individual and to personalize the recommendation of fiber foodstuffs.

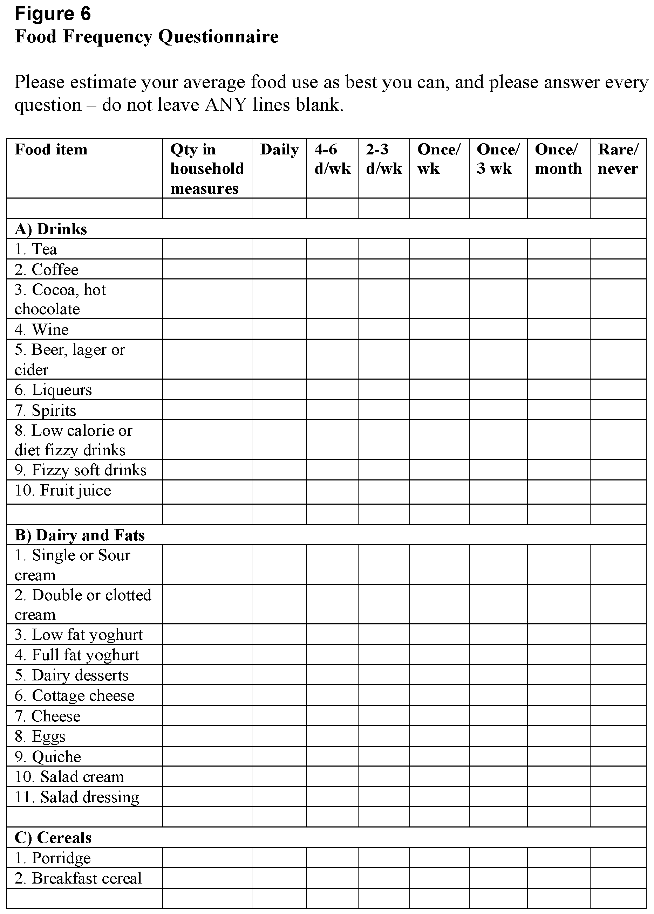

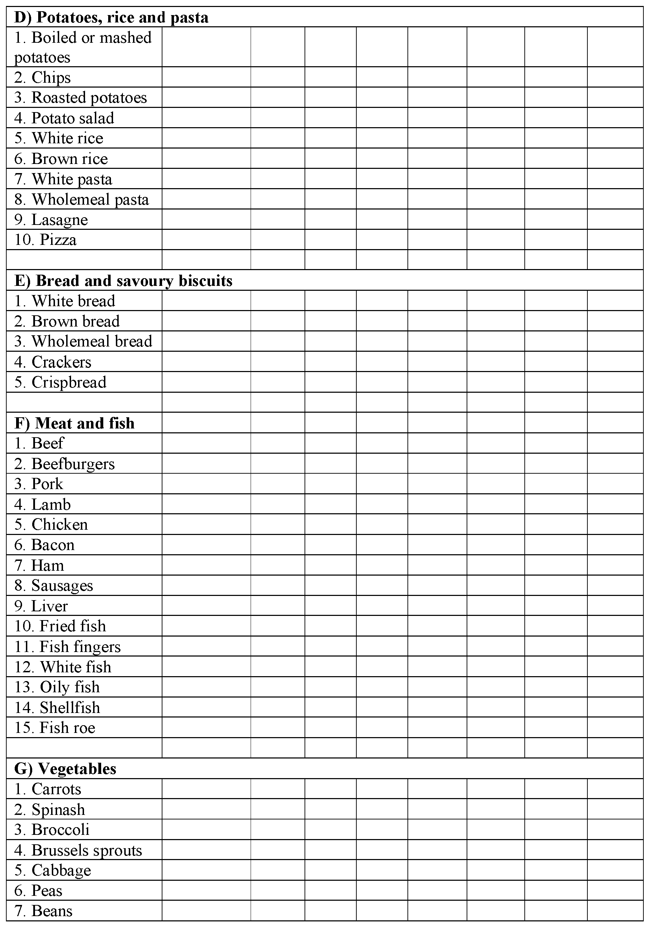

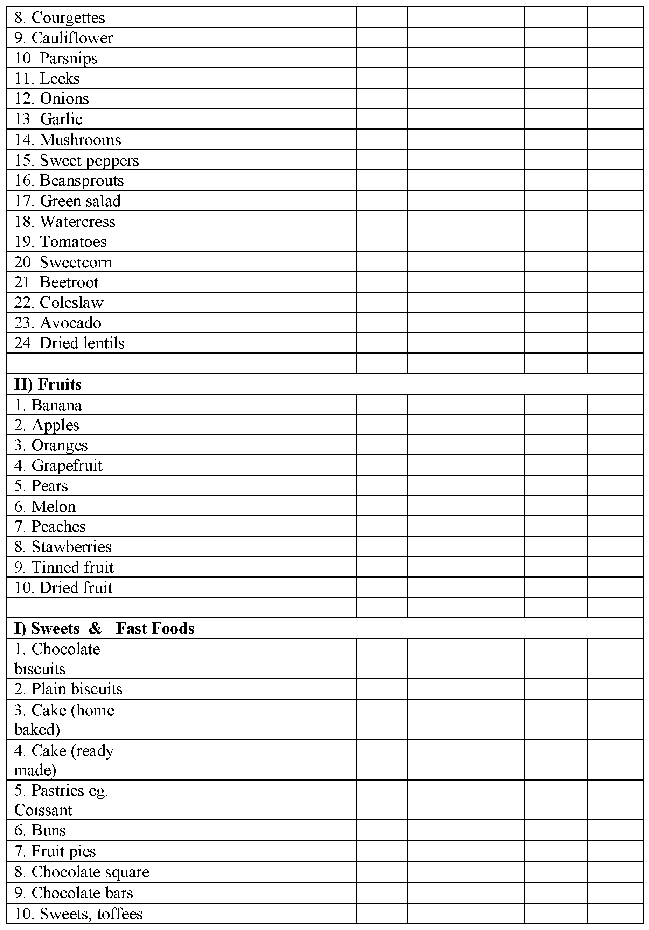

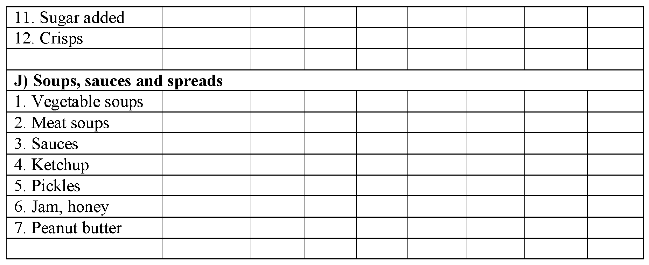

**Figure 6****: Food Frequency Questionnaire**
   This figure provides a typical questionnaire collected from the participants used to determine their CAZyme profile.

### DETAILED DESCRIPTION OF THE INVENTION

All percentages expressed herein are by weight of the total weight of the composition unless expressed otherwise. As used herein, "about," "approximately" and "substantially" are understood to refer to numbers in a range of numerals, for example the range of -10% to +10% of the referenced number, preferably -5% to +5% of the referenced number, more preferably - 1% to +1% of the referenced number, most preferably -0.1% to +0.1% of the referenced number.

All numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used in this invention and the appended claims, the singular forms "a," "an" and "the" include plural referents unless the context clearly dictates otherwise. Thus, for example, reference to "a component" or "the component" includes two or more components.

The words "comprise," "comprises" and "comprising" are to be interpreted inclusively rather than exclusively. Likewise, the terms "include," "including" and "or" should all be construed to be inclusive, unless such a construction is clearly prohibited from the context. Nevertheless, the compositions disclosed herein may lack any element that is not specifically disclosed herein. Thus, a disclosure of an embodiment using the term "comprising" includes a disclosure of embodiments "consisting essentially of" and "consisting of" the components identified. Any embodiment disclosed herein can be combined with any other embodiment disclosed herein.

Where used herein, the terms "example" and "such as," particularly when followed by a listing of terms, are merely exemplary and illustrative and should not be deemed to be exclusive or comprehensive. As used herein, a condition "associated with" or "linked with" another condition means the conditions occur concurrently, preferably means that the conditions are caused by the same underlying condition, and most preferably means that one of the identified conditions is caused by the other identified condition.

The terms "food", "foodstuff", "food product" and "food composition" means a product or composition that is intended for ingestion by an individual such as a human and provides at least one ingredient which provides dietary fiber to the individual.

The term "beverage" or "beverage product" means a liquid product or liquid composition that is intended to be ingested orally by an individual such as a human and provides at least one ingredient that provides dietary fiber to the individual.

The term "dietary supplement" means a product which typically is intended to be ingested orally which provides at least one ingredient which contains dietary fiber. Dietary supplements are recommended when the individual cannot get the recommended amount of daily fiber from a normal diet. Dietary supplements typically contain guar fiber, psyllium, glucomannan or β-glucans.

The compositions of the present disclosure, including the many embodiments described herein, can comprise, consist of, or consist essentially of the elements disclosed herein, as well as any additional or optional ingredients, components, or elements described herein or otherwise useful in a diet.

As used herein, the term "isolated" means removed from one or more other compounds or components with which the compound may otherwise be found, for example as found in nature. For example, "isolated" preferably means that the identified dietary fiber is separated from at least a portion of the other cellular material with which it is typically found in nature. In several embodiments, the amount of dietary fiber is calculated in grams per 100 gram of the foodstuff. For example, an apple contains 2.4 g dietary fiber per 100 g of apple.

"Microbiome" as used herein refers to the genetic content of the communities of microbes that live in and on a subject (e.g., a human subject), both sustainably and transiently, including eukaryotes, archaea, bacteria, and viruses (including bacterial viruses (e.g., phage)), wherein "genetic content" includes genomic DNA, RNA such as ribosomal RNA and messenger RNA, the epigenome, plasmids, and all other types of genetic information. In some embodiments, microbiome specifically refers to genetic content of the communities of microorganisms in a niche.

"Microbiota" as used herein refers to the community of microorganisms that occur (sustainably or transiently) in and on a subject (e.g., a human subject), including eukaryotes, archaea, bacteria, and viruses (including bacterial viruses, e.g. phage). In some embodiments, microbiota specifically refers to the microbial community in a niche.

"Dietary fiber" as used herein refers to foodstuffs, for example, found in the form of fruits, vegetables and cereals, which comprise the human diet. These are sources of carbohydrates that are readily digested by human intestinal enzymes, as well as dietary fibers, which are resistant to digestion and absorption in the human small intestine. Dietary fibers undergo complete or partial microbial fermentation in the large intestine. Most dietary fibers are composed of plant cell wall polysaccharides and the fraction of starch that passes through the small intestine without being broken down (known as resistant starch). These polysaccharides comprise many structurally diverse sugar moieties joined together by glycosidic bonds to form chains and branches. Generally, the more complex the polysaccharide, the more enzymes are required for its breakdown. The human genome encodes, at most, only 17 enzymes for the digestion of food glycans, specifically starch, sucrose and lactose.

"Soluble fiber" as used herein refers to fiber which dissolves in water and forms a gel-like substance in the stomach. Bacteria later break the gel down in the large intestine. Soluble fiber provides some calories to the individual. Soluble fiber provides the following benefits: lowering LDL cholesterol in the blood by affecting how the body absorbs dietary fat and cholesterol slowing absorption of other carbohydrates through digestion, which can help regulate blood sugar levels. Sources of soluble fiber include, for example, beans, fruits, oats, nuts, vegetables.

"Insoluble fiber" as used herein is fiber which does not dissolve in water and passes through the gastrointestinal tract, mostly intact. It does not provide calories. Insoluble fiber helps build bulk in the stool, helping a person pass stool more quickly. It can also help prevent constipation. Sources of insoluble fiber include, for example, fruits, nuts, vegetables, whole grain foods.

The "recommended daily intake of dietary fiber" as used herein is based on a daily diet of 2000 calories. The recommended intake for dietary fiber in a 2,000 calorie diet is approximately 25g per day for adult females and 38 g per day for adult males. Individuals need less fiber after 50 years of age at around 21 g for women and 30 g for men. During pregnancy or breastfeeding, women should aim for at least 28 g per day.

Individuals who are allergic to high fiber foods may find it difficult to get enough fiber from a regular diet and may require fiber dietary supplements in order to reach the recommended daily intake of dietary fiber.

"Adverse side-effects of dietary fiber" as used herein can be the result of increasing dietary fiber too rapidly and/or not personalizing the type of dietary fiber to the individual's CAZyme profile. The adverse side effects can include abdominal pain, bloating, flatulence, constipation and/or diarrhea. In particular, these side effects may occur if a person consumes more than 70 g of fiber a day.

"Carbohydrate-active enzymes" or "CAZymes" as used herein are enzymes that assemble or breakdown oligosaccharides and polysaccharides. The classification of CAZymes is updated continuously in the CAZy database http://www.cazy.org/.

For example, for degradative enzymes, the current classification describes a total of 215 families for 680,000 sequences, a number that increases exponentially due to systematic genome sequencing (Garron et al., 2019). The CAZy families group together enzymes that can have different specificity but share a common fold, a common catalytic machinery and the same mechanism, providing useful predictive power on the orientation of the glycosidic bond cleaved and potential transglycosylation side-reactions.

CAZy classification presently comprises the following families:
Glycosyltransferase (GT) whose function is to catalyse glycoside synthesis
Glycoside Hydrolase (GH) whose function is to catalyse the hydrolysis of the glycosidic linkage of glycosides
Polysaccharide Lyase (PL) whose function is to cleave polysachrides containing uronic acid
Carbohydrate Esterase (CE) whose function is to catalyse the de-O or de-N-acetylation of saccharides
Auxiliary Activity (AA) whose function is to help GH, PL and CE to access the carbohydrates comprising the plant cell wall
Carbohydrate Binding Module (CBM) whose function is to bind to soluble and crystalline carbohydrates to direct the catalytic enzymes (GH or PL) to their substrates
Carbohydrate Binding Module (CBM) whose function is non-catalytic but included due to their association with catalytic modules.

Subfamilies are in subgroups, usually indicated by a number, found within a family that share a more recent ancestor and, that are usually more uniform in molecular function.

"CAZyme profile" and "CAZyme cluster" as used herein refer to the determination of different CAZyme families.

CAZyme families are typically categorized according to substrate usage of the enzymes and comparing the abundance of these categories between individuals having different food intakes. However, this is not always straightforward as many CAZyme families have multiple functions, e.g. some of them can digest fibers of both plant and animal origin.

For example, bacteria of the Bacteroidetes phylum are considered primary degraders of polysaccharides and they are found in all ecosystems investigated. In Bacteroidetes genomes, carbohydrate-degrading enzymes (CAZymes) are arranged in gene clusters termed polysaccharide utilization loci (PULs).

The "CAZyme profile of an individual" as used herein is determined from a dietary intake assessment from the individual subject. In a preferred embodiment, the dietary intake assessment may be in the form of questionnaire, such as Food Frequency Questionnaires (FFQ). It may also comprise diet history questionnaire, short dietary assessment instrument, technology-based tools used in dietary intake assessment or any other tool available in the art. The said food frequency questionnaire (FFQ) refers to a finite list of foods and beverages with response categories to indicate usual frequency of consumption over the time period queried. In particular, it may comprise measuring the consumption of: fruits, vegetables, legumes, whole grains, nuts and seeds, potatoes, dairy products, beverages and snacks to estimate the diversity of fiber consumption within a specific period of time and to determine the CAZyme profile of said individual.

Moreover, it may also comprise measuring the consumption of: whole wheat flour, beans, potatoes, tomatoes, bell peppers, cauliflower, apple, banana, tea, sugar and milk to estimate the diversity of fiber consumption within a specific period of time and to determine the CAZyme profile of said individual.

In an embodiment, the dietary intake assessment tool, in particular the food frequency questionnaire may be adapted to population and geographical specificities.

In a preferred embodiment, the determination of the "CAZyme profile of an individual" may be done by using food data to predict CAZyme cluster or profiles through a supervised machine learning algorithm such as random forest, neural network, linear regression or decision tree. This method of determining the CAZyme profile has the advantage that it does not require a biological sample from the individual subject.

In another embodiment of the invention, the CAZyme profile may be determined from a fecal stool sample. The CAZyme profile may be determined using methods known in the art such as whole shotgun metagenomic sequencing (MGS), target sequencing such as PCR, for example.

MGS is an untargeted method to study the genetic component of a biological sample, in this case, a fecal stool sample. Using high throughput sequencing method all genetic material of a sample is sequenced including those encoding for CAZymes. Sequences are then processed to be annotated using bioinformatic pipelines. This provides information regarding which taxa of the organisms present in the sample and at which abundance and their functionality, for example, as CAZymes. Using MGS, it is possible to quantify genes encoding specific CAZyme functions.

The "CAZyme profile of an individual" may be additionally or alternatively determined from a biological sample from the individual subject. In one preferred embodiment, the CAZyme profile may be determined from a fecal stool sample.

CAZyme profile cluster are samples grouped together according to the similarity of their CAZyme profiles. They can be predicted from CAZyme profile by any clustering methods known in the art. Non-limiting examples of methods of clustering may use unsupervised machine learning algorithm (k-mean, Dirichlet). Profiles can be determined through different annotation pipelines used to annotate the geens that encode for CAZymes such as dbCAN or EggNOG.

"Fiber composition administration" as used herein is typically delivered on a daily basis.

The fiber composition is administered to the individual, at least two days per week, more preferably at least three days per week, most preferably all seven days of the week; for at least one week, at least one month, at least two months, at least three months, at least six months, or even longer. In some embodiments, the composition is administered to the individual consecutively for a number of days. In an embodiment, the composition can be administered to the individual daily for at least 30, 60 or 90 consecutive days. In a preferred embodiment, the dietary fiber composition administration should not exceed 70g fiber per day.

In one embodiment, the fiber composition is preferred to be in the range of 20 g to 28 g per day for women. For women over the age of 50, the fiber composition is preferred to be 21 g per day. For lactating women, the fiber composition is preferred to be 28 g per day.

In one embodiment, the fiber composition is preferred to be in the range of 30 g to 38 g per day for men. For men over the age of 50, the fiber composition is preferred to be 30 g per day.

The above examples of administration do not require continuous daily administration with no interruptions. Instead, there may be some short breaks in the administration, such as a break of two to four days during the period of administration. The ideal duration of the administration of the composition can be determined by those of skill in the art.

In a preferred embodiment, the dietary fiber composition is administered to the individual orally. For example, the composition can be administered to the individual as a food product, a beverage product, and/or dietary supplement.

A "system" as used herein may be illustrated, for example, by Figure 5 which shows an example of a system of a host device 100 usable to implement at least portions of the computerized recommendation system disclosed herein.

In one embodiment, the device 100 illustrated in Figure 5 corresponds to one or more servers and/or other computing devices that provide some or all of the following functions: (a) enabling access to the disclosed system by remote users of the system; (b) serving web page(s) that enable remote users to interface with the disclosed system; (c) storing and/or calculating underlying data, such as recommended fiber intake ranges per gender, recommended fiber consumption ranges, and fiber content of foodstuffs, dietary assessment and/or food frequency questionnaire raw data, CAZyme profile, needed to implement the disclosed system; (d) calculating and displaying component; and/or (e) making recommendations of foodstuffs, food products, beverage products, dietary supplements, menus or recipes or other consumables that can be consumed to help individuals reach an optimal daily personalized fiber intake based on their personal CAZyme profile.

In a preferred embodiment said calculating underlying data can be performed based on CAZyme profiles of the cluster or of the sample as known in the art.

In the example architecture illustrated in Fig. 5, the device 100 includes a main unit 104 which preferably includes one or more processors 106 electrically coupled by an address/data bus 113 to one or more memory devices 108, other computer circuitry 110, and/or one or more interface circuits 112. The one or more processors 106 may be any suitable processor, such as a microprocessor from the INTEL PENTIUM^{®} or INTEL CELERON^{®} family of microprocessors. PENTIUM^{®} and CELERON^{®} are trademarks registered to Intel Corporation and refer to commercially available microprocessors. It should be appreciated that in other embodiments, other commercially-available or specially-designed microprocessors may be used as processor 106. In one embodiment, processor 106 is a system on a chip ("SOC") designed specifically for use in the disclosed system.

In one embodiment, device 100 further includes memory 108. Memory 108 preferably includes volatile memory and non-volatile memory. Preferably, the memory 108 stores one or more software programs that interact with the hardware of the host device 100 and with the other devices in the system as described below. Additionally or alternatively, the programs stored in memory 108 may interact with one or more client devices such as client device 102 (discussed in detail below) to provide those devices with access to media content stored on the device 100. The programs stored in memory 108 may be executed by the processor 106 in any suitable manner.

The interface circuit(s) 112 may be implemented using any suitable interface standard, such as an Ethernet interface and/or a Universal Serial Bus (USB) interface. One or more input devices 114 may be connected to the interface circuit 112 for entering data and commands into the main unit 104. For example, the input device 114 may be a keyboard, mouse, touch screen, track pad, track ball, isopoint, and/or a voice recognition system. In one embodiment, wherein the device 100 is designed to be operated or interacted with only via remote devices, the device 100 may not include input devices 114. In other embodiments, input devices 114 include one or more storage devices, such as one or more flash drives, hard disk drives, solid state drives, cloud storage, or other storage devices or solutions, which provide data input to the host device 100.

One or more storage devices 118 may also be connected to the main unit 104 via the interface circuit 112. For example, a hard drive, CD drive, DVD drive, flash drive, and/or other storage devices may be connected to the main unit 104. The storage devices 118 may store any type of data used by the device 100, including data regarding preferred fiber ranges per gender, data regarding fiber content of various foodstuffs, data regarding users of the system, data regarding previously-generated dietary and/or food frequency questionnaires, data regarding CAZyme profiles per user, and any other appropriate data needed to implement the disclosed system, as indicated by block 150.

In several embodiments, the Recommendation System indicated by block 150 may store different database modules which include: for example, a food module grouped in different food groups such as fruits, vegetables, legumes, whole grains, nuts and seeds, potatoes and dark chocolate; or beverage database module; a menu database module (for example with: breakfast, lunch, dinner and snacks); a recipe database module; a dietary constraints module noting allergies or food sensitivities which may exist.

Alternatively or in addition, storage devices 118 may be implemented as cloud-based storage, such that access to the storage 118 occurs via an internet or other network connectivity circuit such as an Ethernet circuit 112.

One or more displays 120, and/or printers, speakers, or other output devices 119 may also be connected to the main unit 104 via the interface circuit 112. The display 120 may be a liquid crystal display (LCD), a suitable projector, or any other suitable type of display. The display 120 generates visual representations of various data and functions of the host device 100 during operation of the host device 100. For example, the display 120 may be used to display information about the database of preferred dietary fiber ranges, a database of fiber content of various food items, a database of users of the system, a database of previously-generated menus, recipes or meals, and/or databases to enable an administrator at the device 100 to interact with the other databases described above.

In the illustrated embodiment, the users of the computerized recommendation system interact with the device 100 using a suitable client device, such as client device 102. The client device 102 in various embodiments is any device that can access content provided or served by the host device 100. For example, the client device 102 may be any device that can run a suitable web browser to access a web-based interface to the host device 100. Alternatively or in addition, one or more applications or portions of applications that provide some of the functionality described herein may operate on the client device 102, in which case the client device 102 is required to interface with the host device 100 merely to access data stored in the host device 100, such as data regarding recommended daily dietary fiber ranges or fiber content of various food items.

In one embodiment, this connection of devices (i.e., the device 100 and the client device 102) is facilitated by a network connection over the Internet and/or other networks, illustrated in Figure 5 by cloud 116. The network connection may be any suitable network connection, such as an Ethernet connection, a digital subscriber line (DSL), a WiFi connection, a cellular data network connection, a telephone line-based connection, a connection over coaxial cable, or another suitable network connection.

In one embodiment, host device 100 is a device that provides cloud-based services, such as cloud-based authentication and access control, storage, streaming, and feedback provision. In this embodiment, the specific hardware details of host device 100 are not important to the implementer of the disclosed system-instead, in such an embodiment, the implementer of the disclosed system utilizes one or more Application Programmer Interfaces (APIs) to interact with host device 100 in a convenient way, such as to enter information about the user's demographics to help determine dietary fiber ranges, for example, based on gender, to enter information about consumed foods, and other interactions described in more detail below.

Access to device 100 and/or client device 102 may be controlled by appropriate security software or security measures. An individual user's access can be defined by the device 100 and limited to certain data and/or actions, such as selecting or viewing total dietary fiber consumption over a day or other time period, according to the individual's identity. Other users of either host device 100 or client device 102 may be allowed to alter other data, depending on those users' identities. Accordingly, users of the system may be required to register with the device 100 before accessing the content provided by the disclosed system.

In a preferred embodiment, each client device 102 has a similar structural or architectural makeup to that described above with respect to the device 100. That is, each client device 102 in one embodiment includes a display device, at least one input device, at least one memory device, at least one storage device, at least one processor, and at least one network interface device. It should be appreciated that by including such components, which are common to well-known desktop, laptop, or mobile computer systems (including smart phones, tablet computers, and the like), client device 102 facilitates interaction among and between each other by users of the respective systems.

In various embodiments, devices 100 and/or 102 as illustrated in Figure 5 may in fact be implemented as a plurality of different devices. For example, the device 100 may in actuality be implemented as a plurality of server devices operating together to implement the media content access system described herein. In various embodiments, one or more additional devices, not shown in Figure. 5, interact with the device 100 to enable or facilitate access to the system disclosed herein. For example, in one embodiment the host device 100 communicates via network 116 with one or more public, private, or proprietary repositories of information, such as public, private, or proprietary repositories of CAZyme information, dietary fiber content information, menu planners, recipe databases, energy information, environmental impact information, or the like.

In one embodiment, the disclosed system does not include a client device 102. In this embodiment, the functionality described herein is provided on host device 100, and the user of the system interacts directly with host device 100 using input devices 114, display device 120, and output devices 119. In this embodiment, the host device 100 provides some or all of the functionality described herein as being user-facing functionality.

In various embodiments, the system disclosed herein is arranged as a plurality of modules, wherein each module performs a particular function or set of functions. The modules in these embodiments could be software modules executed by a general purpose processor, software modules executed by a special purpose processor, firmware modules executing on an appropriate, special-purpose hardware device, or hardware modules (such as application specific integrated circuits ("ASICs")) that perform the functions recited herein entirely with circuitry. In embodiments where specialized hardware is used to perform some or all of the functionality described herein, the disclosed system may use one or more registers or other data input pins to control settings or adjust the functionality of such specialized hardware.

For example, the system includes: an input module for determining the CAZyme profile of an individual; a calculation module for calculating the relationship of the CAZyme profile of said individual to dietary fiber intake; and a recommendation module for recommending dietary fiber compositions for an individual. Additionally, the system can provide an output module for delivering the personalized dietary fiber composition.

A user goal to personalize the dietary fiber recommendation based on the CAZyme profile of the individual user. The system can be used to provide food products, beverage products, dietary supplements, as well as menus or recipes in order to get closer to the recommended amounts of daily fiber. In some embodiments, the system and methods disclosed herein can be used by nutritionists, health-care professionals, and individual users (e.g., users of wearable devices such as smart watches or fitness trackers).

### EXAMPLES

### Example 1: Identification of CAZyme profile and CAZyme clusters

We used data collected from 60 volunteers that reported their dietary habits through food frequency questionnaires (FFQ) and provided faecal samples for CAZyme profiling via whole shotgun metagenomics sequencing MGS.

The CAZyme profile was determined from a food frequency questionnaire from the individual subject as the composition of the gut microbiota and its metabolic capacity was predominately shaped by diet (Figure 5). Food consumed provided substrates to the ecosystem and conditions which encourage the flourishing of defined taxa. Consequently, by evaluating the foodstuffs and specifically fiber content in the foodstuffs, we could predict the likelihood of the presence of certain bacteria and their function adapted to substrate availability.

The food frequency questionnaires were used in tandem with a machine learning algorithm to determine the likelihood of an individual to present a specific CAZyme profile. This method of determining the CAZyme profile had the advantage that it does not require a biological sample each time from the individual subject once the model has been built.

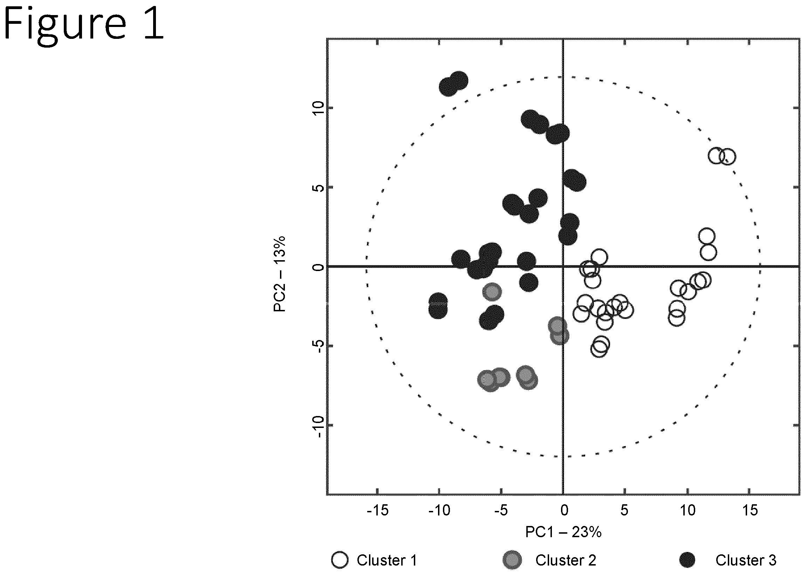

The food frequency questionnaires were then used to assign individuals to different CAZyme clusters (Figures 1, 2, 3, 4) Hence, CAZyme clusters were predefined using faecal samples from a cohort used to build the initial model.

CAZyme profiling was used to infer the gut microbiome capacity to degrade dietary fibers from analysis of faecal samples. CAZyme profile from fecal sample was assessed using whole shotgun metagenomic sequencing (MGS). Using a high throughput sequencing method all genetic material of a fecal sample from individuals was sequenced, especially those encoding for CAZymes. Sequences were then processed to be annotated using bioinformatic pipelines which provided information regarding which taxa (organisms) were present in the sample and at which abundance but also identify and group CAZymes that code for a specific function.

Raw CAZyme copy numbers were normalised by the total number of CAZymes and scaled. Clusters of CAZyme profiles were then defined using a k mean algorithm.

We used a Random Forest algorithm to predict CAZyme clusters with the FFQs data (non-transformed to nutrient intake). To build the models, we randomly selected 50 times 40 samples to generate the training set and 20 for the test set. The performance of the models was evaluated by calculating the area under the curve (AUC) of the ROC curves.

### Results:

A total of three clusters based on CAZyme profiles were identified in the population (Figures 1,2,3,4). Each cluster was defined by a specific CAZyme profile that may reflect the gut microbiome capability to degrade dietary fibers. After optimisation, food frequency questionnaires could be used to predict CAZyme clusters with a mean AUC = 0.79. A total of 11 foods, selected during the optimisation process, were used to build these models (Figure 4).

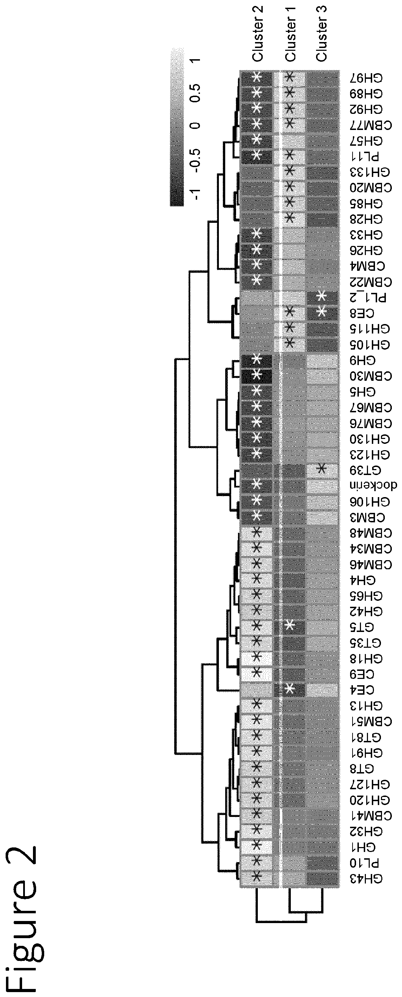

Further investigation of the role of the CAZymes defining each cluster revealed that this information could be used to determine which carbohydrate could be degraded by the microbiome of the sub-population (Figure 2). As an illustration, we observed that cluster 1 was enriched in CAZymes able to degrade pectin and pectic-like structure (rhamnogalacturonan I - RGI) [Cecchini et al., 2013], namely CE8, GH28, GH 105 and PL11 and for which cauliflower (that contains pectin and RGI) was the most important predictor. Second, in cluster 2, we observed an enrichment in CAZymes involved in the fermentation of arabinogalactan and arabinan (GH43) that are found in bell pepper, which is one of the main food predictors for this cluster. In Cluster 3, we observed a combination between CAZymes profiles different from those in Clusters 1 and 2. Hence, in-depth analysis of the CAZymes enriched in each cluster can be used to determine which fiber type or fiber blend will be the most likely to be utilised by the microbiome and therefore to elicit beneficial effects on the host.

### Conclusion:

We demonstrated that clusters of CAZyme profiles can be predicted based on dietary intake. Additionally, analysis of CAZymes enriched in each cluster can help to design a fiber composition that should be optimally utilised by the individual subject.

### References

Bhattacharya, T., Ghosh, T. S., & Mande, S. S. (2015). Global profiling of carbohydrate active enzymes in human gut microbiome. PloS one, 10(11), e0142038.
Cecchini, D. A., Laville, E., Laguerre, S., Robe, P., Leclerc, M., Dore, J., & Potocki-Véronèse, G. (2013). Functional metagenomics reveals novel pathways of prebiotic breakdown by human gut bacteria. PloS one, 8(9), e72766.
Flint, H. J., Scott, K. P., Duncan, S. H., Louis, P., & Forano, E. (2012). Microbial degradation of complex carbohydrates in the gut. Gut microbes, 3(4), 289-306.
Garron, M. L., & Henrissat, B. (2019). The continuing expansion of CAZymes and their families. Current opinion in chemical biology, 53, 82-87.
Kaur, K., Khatri, I., Akhtar, A., Subramanian, S., & Ramya, T. N. C. (2020). Metagenomics analysis reveals features unique to Indian distal gut microbiota. PloS one, 15(4), e0231197.
Kovatcheva-Datchary, P., Nilsson, A., Akrami, R., Lee, Y. S., De Vadder, F., Arora, T., & Bäckhed, F. (2015). Dietary fiber-induced improvement in glucose metabolism is associated with increased abundance of Prevotella. Cell metabolism, 22(6), 971-982.
Makki, K., Deehan, E. C., Walter, J., & Bäckhed, F. (2018). The impact of dietary fiber on gut microbiota in host health and disease. Cell host & microbe, 23(6), 705-715.
Threapleton, D. E., Greenwood, D. C., Evans, C. E., Cleghorn, C. L., Nykjaer, C., Woodhead, C., & Burley, V. J. (2013). Dietary fiber intake and risk of cardiovascular disease: systematic review and meta-analysis. Bmj, 347, f6879.

## Claims

1. A computer-implemented method for providing a personalized fiber recommendation to an individual wherein said method comprises
(i) determining a carbohydrate-active enzyme, CAZyme, profile of said individual, CAZymes being enzymes that assemble or breakdown oligosaccharides and polysaccharides, and
(ii) providing a recommendation of a personalized fiber composition,
wherein the CAZyme profile of said individual is determined by
using a dietary assessment tool for assessing a dietary intake of said individual, the dietary assessment tool comprising diet history questionnaire, short dietary assessment instrument, food frequency questionnaire or technology based tools used in dietary intake assessment, and,
assigning said individual to a CAZyme profile cluster of different CAZyme profile clusters using the assessed dietary intake of said individual, the different CAZyme profile clusters being samples grouped together according to a similarity of their CAZymes profiles and the different CAZyme profile clusters being predefined using fecal samples and dietary habits of volunteers, and
wherein the recommended personalized fiber composition is designed by analyzing CAZymes enriched in the CAZyme profile cluster to which said individual is assigned.

2. The computer-implemented method according to claim 1, wherein said dietary assessment tool comprises measuring the consumption of: fruits, vegetables, legumes, whole grains, nuts and seeds, potatoes, dairy products, beverages and snacks to estimate the diversity of fiber consumption within a specific period of time and to determine the CAZyme profile of said individual.

3. The computer-implemented method according to claim 2, wherein said dietary assessment tool comprises measuring the consumption of: whole wheat flour, beans, potatoes, tomatoes, bell peppers, cauliflower, apple, banana, tea, sugar and milk to estimate the diversity of fiber consumption within a specific period of time and to determine the CAZyme profile of said individual.

4. The computer-implemented method according to any one of claims 1 to 3 wherein the CAZyme profile of said individual is additionally determined by a biological sample from said individual, wherein the biological sample is a fecal sample.

5. The computer-implemented method according to any one of claims 1 to 4 wherein the personalized fiber recommendation is a daily fiber intake less than 70 g per day and determined by the gender of the individual.

6. The computer-implemented method according to claim 5 wherein the daily fiber recommendation is preferably between 20 g to 28 g per day for females and not exceeding 70 g per day.

7. The computer-implemented method according to claim 5 wherein the daily fiber recommendation is preferably between 30 g to 38 g per day for males and not exceeding 70 g per day.

8. The computer-implemented method according to any one of claims 1 to 7 wherein the personalized fiber recommendation is preferably fiber in the form of consumable dietary fiber.

9. The computer-implemented method according to claim 8 wherein the personalized fiber recommendation is a food product, beverage product, or dietary supplement containing a high fiber foodstuff.

10. The computer-implemented method according to claim 9 wherein the personalized fiber recommendation in the form of consumable dietary fiber is selected from high fiber foodstuffs comprising: fruits, vegetables, legumes, whole grains, nuts and seeds, potatoes with skin and dark chocolate in an amount not exceeding 70g of fiber per day and personalized for females in the amount between 20 g to 28 g fiber per day and for males in the amount between 30 g to 38 g fiber per day.

11. The computer-implemented method according to claim 10 wherein the high fiber foodstuffs comprising fruits containing fiber in the amount of at least about 2.0 g to 10.0 g per 100g are selected from the group comprising: passionfruit 10.0 g/100g; avocado 6.7 g/100g; raspberries 6.5 g/100g; blackberries 5.3g/100g; guava 5.0 g/100g; persimmon 4.5 g/100g; mango 3.5 g/100g; pears 3.1 g/100g; banana 2.6 g/100g; apples 2.4 g/100g; blueberries 2.4 g/100g; or strawberries 2.0 g/100g.

12. The computer-implemented method according to claim 10 wherein the high fiber foodstuffs comprising vegetables containing fiber in the amount of at least about 1.0 g to 10.0 g per 100 g are selected from the group comprising: artichokes 8.6 g/100g; kale 3.6 g/100g; carrots 2.8 g/100g; beets 2.8 g/100g; broccoli 2.6 g/100g; Brussel sprouts 2.6 g/100g; spinach 2.2 g/100g; cauliflower 2.0 g/100g; bell peppers 1.2 g/100g or tomatoes 1.2 g/100g.

13. The computer-implemented method according to claim 10 wherein the high fiber foodstuffs comprising legumes containing fiber in the amount of at least about 5.0 g to 9.0 g per 100g are selected from the group comprising: black beans 8.7 g/100g; split peas 8.3 g/100g; lentils 7.9 g/100g; chickpeas 7.6 g/100g; kidney beans 6.4 g/100g; baked beans 5.5 g/100g; lima beans 5.3 g/100g; or edamame beans 5.2 g/100g.

14. The computer-implemented method according to claim 10 wherein the high fiber foodstuffs comprising whole grains containing fiber in the amount of at least about 2.8 g to 14.5 g per 100g are selected from the group comprising: barley 17.0g/100g; whole grain flour 11 g/100g; oats 10.6 g/100g; quinoa 2.8 g/100g; or popcorn 14.5 g/100g.

15. The computer-implemented method according to claim 10 wherein the high fiber foodstuffs comprising nuts and seeds containing fiber in the amount of at least about 7.0 g to 35.5 g per 100g are selected from the group comprising: chia seeds 34.4g/100g; pumpkin seeds 18.4 g/100g; almonds 12.5 g/100g; pistachios 10.0 g/100g; coconut 9.0 g/100g; sunflower seeds 8.6 g/100g; or walnuts 7.0 g/100g.

16. The computer-implemented method according to claim 10 wherein the high fiber foodstuffs comprising potatoes with skin containing fiber in the amount of at least about 2.5g per 100g.

17. The computer-implemented method according to claim 10 wherein the high fiber foodstuffs comprising dark chocolate containing at least 70% cocoa containing fiber in the amount of at least about 10.9 g per 100g.

18. The computer-implemented method according to claim 9 wherein the personalized fiber recommendation is in the form of consumable dietary fiber and a fiber supplement.

19. The computer-implemented method according to claim 18 wherein the personalized fiber recommendation of a fiber supplement is selected from a supplement containing guar fiber, psyllium, glucomannan or β-glucans.

20. The computer-implemented method according to any one of claims 1 to 19 comprising: (iii) delivering the personalized fiber composition.

21. A system for providing a personalized fiber recommendation to an individual comprising:
(i) an input module for determining a carbohydrate-active enzyme, CAZyme, profile of said individual, CAZymes being enzymes that assemble or breakdown oligosaccharides and polysaccharides;
(ii) a calculation module for calculating the relationship of the CAZyme profile of said individual to fiber intake; and
(iii) a recommendation module for recommending a personalized fiber composition for said individual, wherein
the system is configured to
use a dietary assessment tool for assessing a dietary intake of said individual, the dietary assessment tool comprising diet history questionnaire, short dietary assessment instrument, food frequency questionnaire or technology based tools used in dietary intake assessment,
assign said individual to a CAZyme profile cluster of different CAZyme profile clusters using the assessed dietary intake of said individual, the different CAZyme profile clusters being samples grouped together according to a similarity of their CAZymes profiles and the different CAZyme profile clusters being predefined using fecal samples and dietary habits of volunteers, and
design the recommended personalized fiber composition by analyzing CAZymes enriched in the CAZyme profile cluster to which said individual is assigned.

22. The system according to claim 21 further comprising:
(iv) an output module for delivering the personalized fiber composition.

23. The system according to claim 21 or 22 wherein the personalized fiber composition is selected from the group consisting of: food products, beverage products, or dietary supplements or a combination thereof in a kit of parts delivered to the individual.

## Patentansprüche

1. Computerimplementiertes Verfahren zur Bereitstellung einer personalisierten Ballaststoffempfehlung für eine Person, wobei das Verfahren umfasst
(i) Bestimmen eines kohlenhydrataktiven Enzymprofils, CAZym-Profils, der Person, wobei CAZyme Enzyme sind, die Oligosaccharide und Polysaccharide aufbauen oder abbauen, und
(ii) Bereitstellen einer Empfehlung für eine personalisierte Ballaststoffzusammensetzung,
wobei das CAZym-Profil der Person bestimmt wird durch
Verwenden eines Instruments zur Ernährungsbewertung, um die Nahrungsaufnahme der betreffenden Person zu bewerten, wobei das Instrument zur Ernährungsbewertung einen Fragebogen zur Ernährungshistorie, ein kurzes Instrument zur Ernährungsbewertung, einen Fragebogen zur Häufigkeit der Nahrungsaufnahme oder technologiebasierte Instrumente zur Bewertung der Nahrungsaufnahme umfasst, und
Zuordnen der Person zu einem CAZym-Profilcluster aus verschiedenen CAZym-Profilclustern anhand der bewerteten Nahrungsaufnahme der Person, wobei die verschiedenen CAZym-Profilcluster Proben sind, die entsprechend der Ähnlichkeit ihrer CAZyme-Profile gruppiert sind, und die verschiedenen CAZym-Profilcluster anhand von Stuhlproben und Ernährungsgewohnheiten von Freiwilligen vordefiniert sind, und
wobei die empfohlene personalisierte Ballaststoffzusammensetzung durch die Analyse von CAZymen erstellt wird, die im CAZym-Profilcluster angereichert sind, dem die Person zugeordnet ist.

2. Computerimplementiertes Verfahren nach Anspruch 1, wobei das Instrument zur Ernährungsbewertung die Messung des Verzehrs von: Obst, Gemüse, Hülsenfrüchten, Vollkornprodukten, Nüssen und Samen, Kartoffeln, Milchprodukten, Getränken und Snacks umfasst, um die Vielfalt des Ballaststoffkonsums innerhalb eines bestimmten Zeitraums abzuschätzen und das CAZym-Profil der Person zu bestimmen.

3. Computerimplementiertes Verfahren nach Anspruch 2, wobei das Instrument zur Ernährungsbewertung die Messung des Verzehrs von: Vollkornmehl, Bohnen, Kartoffeln, Tomaten, Paprika, Blumenkohl, Äpfeln, Bananen, Tee, Zucker und Milch umfasst, um die Vielfalt des Ballaststoffkonsums innerhalb eines bestimmten Zeitraums abzuschätzen und das CAZym-Profil der Person zu bestimmen.

4. Computerimplementiertes Verfahren nach einem der Ansprüche 1 bis 3, wobei das CAZym-Profil der Person zusätzlich durch eine biologische Probe der Person bestimmt wird, wobei die biologische Probe eine Stuhlprobe ist.

5. Computerimplementiertes Verfahren nach einem der Ansprüche 1 bis 4, wobei die personalisierte Ballaststoffempfehlung eine tägliche Ballaststoffaufnahme von weniger als 70 g pro Tag ist und durch das Geschlecht der Person bestimmt wird.

6. Computerimplementiertes Verfahren nach Anspruch 5, wobei die tägliche Ballaststoffempfehlung für Frauen vorzugsweise zwischen 20 g und 28 g pro Tag liegt und 70 g pro Tag nicht übersteigt.

7. Computerimplementiertes Verfahren nach Anspruch 5, wobei die tägliche Ballaststoffempfehlung für Männer vorzugsweise zwischen 30 g und 38 g pro Tag liegt und 70 g pro Tag nicht übersteigt.

8. Computerimplementiertes Verfahren nach einem der Ansprüche 1 bis 7, wobei die personalisierte Ballaststoffempfehlung vorzugsweise Ballaststoffe in Form von verzehrbaren Ballaststoffen umfasst.

9. Computerimplementiertes Verfahren nach Anspruch 8, wobei die personalisierte Ballaststoffempfehlung ein Nahrungsmittel, ein Getränkeprodukt oder ein Nahrungsergänzungsmittel ist, das ein ballaststoffreiches Nahrungsmittel enthält.

10. Computerimplementiertes Verfahren nach Anspruch 9, wobei die personalisierte Ballaststoffempfehlung in Form von verzehrbaren Ballaststoffen aus ballaststoffreichen Nahrungsmitteln ausgewählt wird, umfassend: Obst, Gemüse, Hülsenfrüchte, Vollkornprodukte, Nüsse und Samen, Kartoffeln mit Schale und dunkle Schokolade in einer Menge von nicht mehr als 70 g Ballaststoffen pro Tag und personalisiert für Frauen in einer Menge zwischen 20 g und 28 g Ballaststoffen pro Tag und für Männer in einer Menge zwischen 30 g und 38 g Ballaststoffen pro Tag.

11. Computerimplementiertes Verfahren nach Anspruch 10, wobei die ballaststoffreichen Nahrungsmittel, die Obst umfassen, das Ballaststoffe in einer Menge von mindestens etwa 2,0 g bis 10,0 g pro 100 g enthält, aus der Gruppe ausgewählt werden, umfassend: Passionsfrucht 10,0 g/100 g; Avocado 6,7 g/100 g; Himbeeren 6,5 g/100 g; Brombeeren 5,3 g/100 g; Guave 5,0 g/100 g; Kaki 4,5 g/100 g; Mango 3,5 g/100 g; Birnen 3,1 g/100 g; Banane 2,6 g/100 g; Äpfel 2,4 g/100 g; Heidelbeeren 2,4 g/100 g; oder Erdbeeren 2,0 g/100 g.

12. Computerimplementiertes Verfahren nach Anspruch 10, wobei die ballaststoffreichen Nahrungsmittel, die Gemüse umfassen, das Ballaststoffe in einer Menge von mindestens etwa 1,0 g bis 10,0 g pro 100 g enthält, aus der Gruppe ausgewählt werden, umfassend: Artischocken 8,6 g/100 g; Grünkohl 3,6 g/100 g; Karotten 2,8 g/100 g; Rüben 2,8 g/100 g; Brokkoli 2,6 g/100 g; Rosenkohl 2,6 g/100 g; Spinat 2,2 g/100 g; Blumenkohl 2,0 g/100 g; Paprika 1,2 g/100 g oder Tomaten 1,2 g/100 g.

13. Computerimplementiertes Verfahren nach Anspruch 10, wobei die ballaststoffreichen Nahrungsmitteldie Hülsenfrüchte umfassen, die Ballaststoffe in einer Menge von mindestens etwa 5,0 g bis 9,0 g pro 100 g enthalten, aus der Gruppe ausgewählt werden, umfassend: schwarze Bohnen 8,7 g/100 g; Spalterbsen 8,3 g/100 g; Linsen 7,9 g/100 g; Kichererbsen 7,6 g/100 g; Kidneybohnen 6,4 g/100 g; gebackene Bohnen 5,5 g/100 g; Limabohnen 5,3 g/100 g; oder Edamame-Bohnen 5,2 g/100 g.

14. Computerimplementiertes Verfahren nach Anspruch 10, wobei die ballaststoffreichen Nahrungsmittel, die Vollkornprodukte umfassen, die Ballaststoffe in einer Menge von mindestens etwa 2,8 g bis 14,5 g pro 100 g enthalten, aus der Gruppe ausgewählt werden, umfassend: Gerste 17,0 g/100 g; Vollkornmehl 11 g/100g; Hafer 10,6 g/100 g; Quinoa 2,8 g/100 g; oder Popcorn 14,5 g/100 g.

15. Computerimplementiertes Verfahren nach Anspruch 10, wobei die ballaststoffreichen Nahrungsmittel, die Nüsse und Samen umfassen, die Ballaststoffe in einer Menge von mindestens etwa 7,0 g bis 35,5 g pro 100 g enthalten, aus der Gruppe ausgewählt werden, umfassend: Chiasamen 34,4 g/100 g; Kürbiskerne 18,4 g/100 g; Mandeln 12,5 g/100 g; Pistazien 10,0 g/100 g; Kokosnuss 9,0 g/100 g; Sonnenblumenkerne 8,6 g/100 g; oder Walnüsse 7,0 g/100 g.

16. Computerimplementiertes Verfahren nach Anspruch 10, wobei die ballaststoffreichen Nahrungsmittel Kartoffeln mit Schale umfassen, die Ballaststoffe in einer Menge von mindestens etwa 2,5 g pro 100 g enthalten.

17. Computerimplementiertes Verfahren nach Anspruch 10, wobei die ballaststoffreichen Nahrungsmittel dunkle Schokolade umfassen, die mindestens 70 % Kakao enthält, der Ballaststoffe in einer Menge von mindestens etwa 10,9 g pro 100 g enthält.

18. Computerimplementiertes Verfahren nach Anspruch 9, wobei die personalisierte Ballaststoffempfehlung in Form von verzehrbaren Ballaststoffen und einem Ballaststoffzusatz erfolgt.

19. Computerimplementiertes Verfahren nach Anspruch 18, wobei die personalisierte Ballaststoffempfehlung eines Ballaststoffpräparats aus einem Präparat ausgewählt wird, das Guarfasern, Psyllium, Glucomannan oder β-Glucane enthält.

20. Computerimplementiertes Verfahren nach einem der Ansprüche 1 bis 19, umfassend:
(iii) Bereitstellung der personalisierten Ballaststoffzusammensetzung.

21. System zur Bereitstellung einer personalisierten Ballaststoffempfehlung für eine Person, umfassend:
(i) ein Eingabemodul zum Bestimmen eines kohlenhydrataktiven Enzymprofils, CAZym-Profils, der Person, wobei CAZyme Enzyme sind, die Oligosaccharide und Polysaccharide aufbauen oder abbauen;
(ii) ein Berechnungsmodul zum Berechnen der Beziehung des CAZym-Profils der Person zur Ballaststoffaufnahme; und
(iii) ein Empfehlungsmodul zum Empfehlen einer personalisierten Ballaststoffzusammensetzung für die Person, wobei
das System konfiguriert ist zum
Verwenden eines Instruments zur Ernährungsbewertung, um die Nahrungsaufnahme der betreffenden Person zu bewerten, wobei das Instrument zur Ernährungsbewertung einen Fragebogen zur Ernährungshistorie, ein kurzes Instrument zur Ernährungsbewertung, einen Fragebogen zur Häufigkeit der Nahrungsaufnahme oder technologiebasierte Instrumente zur Bewertung der Nahrungsaufnahme umfasst,
Zuordnen der Person zu einem CAZym-Profilcluster aus verschiedenen CAZym-Profilclustern anhand der bewerteten Nahrungsaufnahme der Person, wobei die verschiedenen CAZym-Profilcluster Proben sind, die entsprechend der Ähnlichkeit ihrer CAZyme-Profile gruppiert sind, und die verschiedenen CAZym-Profilcluster anhand von Stuhlproben und Ernährungsgewohnheiten von Freiwilligen vordefiniert sind, und
Entwerfen der empfohlenen personalisierten
Ballaststoffzusammensetzung durch Analysieren der CAZyme, die im CAZym-Profilcluster angereichert sind, dem die jeweilige Person zugeordnet ist.

22. Verfahren nach Anspruch 21, ferner umfassend:
(iv) ein Ausgabemodul zur Bereitstellung der personalisierten Ballaststoffzusammensetzung.

23. System nach Anspruch 21 oder 22, wobei die personalisierte Ballaststoffzusammensetzung ausgewählt ist aus der Gruppe bestehend aus: Nahrungsmittelprodukten, Getränkeprodukten oder Nahrungsergänzungsmitteln oder einer Kombination davon in einem an die Person gelieferten Teilesatz.

## Revendications

1. Procédé mis en œuvre par ordinateur pour la fourniture d'une recommandation de fibres personnalisée à un individu, dans lequel ledit procédé comprend
(i) la détermination d'un profil d'enzymes actives sur les glucides, CAZyme, dudit individu, les CAZymes étant des enzymes qui assemblent ou décomposent des oligosaccharides et des polysaccharides, et
(ii) la fourniture d'une recommandation d'une composition de fibres personnalisée,
dans lequel le profil CAZyme dudit individu est déterminé par
l'utilisation d'un outil d'évaluation d'alimentation pour évaluer un apport alimentaire dudit individu, l'outil d'évaluation d'alimentation comprenant un questionnaire concernant des antécédents alimentaires, un instrument d'évaluation d'alimentation court, un questionnaire concernant une fréquence alimentaire ou des outils technologiques utilisés dans une évaluation d'apport alimentaire, et,
l'affectation dudit individu à un groupe de profils CAZyme de différents groupes de profils CAZyme à l'aide de l'apport alimentaire évalué dudit individu, les différents groupes de profils CAZyme étant des échantillons regroupés en fonction d'une similarité de leurs profils CAZymes et les différents groupes de profils CAZyme étant prédéfinis à l'aide d'échantillons fécaux et d'habitudes alimentaires de volontaires, et
dans lequel la composition de fibres personnalisée et recommandée est conçue en analysant des CAZymes enrichis dans le groupe de profils CAZyme auquel ledit individu est affecté.

2. Procédé mis en œuvre par ordinateur selon la revendication 1, dans lequel ledit outil d'évaluation d'alimentation comprend la mesure de la consommation de : fruits, légumes, légumineuses, céréales complètes, fruits à coque et graines, pommes de terre, produits laitiers, boissons et collations, afin d'estimer la diversité de consommation de fibres au cours d'une période de temps spécifique et de déterminer le profil CAZyme dudit individu.

3. Procédé mis en œuvre par ordinateur selon la revendication 2, dans lequel ledit outil d'évaluation d'alimentation comprend la mesure de la consommation de : farine de blé complet, haricots, pommes de terre, tomates, poivrons, chou-fleur, pomme, banane, thé, sucre et lait, afin d'estimer la diversité de consommation de fibres au cours d'une période de temps spécifique et de déterminer le profil CAZyme dudit individu.

4. Procédé mis en œuvre par ordinateur selon l'une quelconque des revendications 1 à 3, dans lequel le profil CAZyme dudit individu est en outre déterminé par un échantillon biologique dudit individu, dans lequel l'échantillon biologique est un échantillon fécal.

5. Procédé mis en œuvre par ordinateur selon l'une quelconque des revendications 1 à 4, dans lequel la recommandation de fibres personnalisée est un apport en fibres quotidien inférieur à 70 g par jour et déterminé par le genre de l'individu.

6. Procédé mis en œuvre par ordinateur selon la revendication 5, dans lequel la recommandation de fibres quotidienne est de préférence entre 20 g à 28 g par jour pour les femmes et n'excède pas 70 g par jour.

7. Procédé mis en œuvre par ordinateur selon la revendication 5, dans lequel la recommandation de fibres quotidienne est de préférence entre 30 g à 38 g par jour pour les hommes et n'excède pas 70 g par jour.

8. Procédé mis en œuvre par ordinateur selon l'une quelconque des revendications 1 à 7, dans lequel la recommandation de fibres personnalisée est de préférence des fibres sous forme de fibres alimentaires consommables.

9. Procédé mis en œuvre par ordinateur selon la revendication 8, dans lequel la recommandation de fibres personnalisée est un produit alimentaire, un produit à boire ou un complément alimentaire contenant une denrée alimentaire riche en fibres.

10. Procédé mis en œuvre par ordinateur selon la revendication 9, dans lequel la recommandation de fibres personnalisée sous forme de fibres alimentaires consommables est choisie parmi des denrées alimentaires riches en fibres comprenant : fruits, légumes, légumineuses, céréales complètes, fruits à coque et graines, pommes de terre avec la peau et chocolat noir dans une quantité n'excédant pas 70 g de fibres par jour et personnalisée pour les femmes dans une quantité entre 20 g à 28 g de fibres par jour et pour les hommes dans une quantité entre 30 g à 38 g de fibres par jour.

11. Procédé mis en œuvre par ordinateur selon la revendication 10, dans lequel les denrées alimentaires riches en fibres comprenant des fruits contenant des fibres dans une quantité d'au moins environ 2,0 g à 10,0 g par 100 g sont choisies dans le groupe comprenant : fruit de la passion 10,0 g/100 g ; avocat 6,7 g/100 g ; framboises 6,5 g/100 g ; mûres 5,3 g/100g ; goyave 5,0 g/100 g ; kaki 4,5 g/100 g ; mangue 3,5 g/100 g ; poires 3,1 g/100 g ; banane 2,6 g/100 g ; pommes 2,4 g/100 g ; myrtilles 2,4 g/100 g ; ou fraises 2,0 g/100 g.

12. Procédé mis en œuvre par ordinateur selon la revendication 10, dans lequel les denrées alimentaires riches en fibres comprenant des légumes contenant des fibres dans une quantité d'au moins environ 1,0 g à 10,0 g par 100 g sont choisies dans le groupe comprenant : artichauts 8,6 g/100 g ; chou frisé 3,6 g/100 g ; carottes 2,8 g/100 g ; betteraves 2,8 g/100 g ; brocoli 2,6 g/100 g ; choux de Bruxelles 2,6 g/100 g ; épinard 2,2 g/100 g ; chou-fleur 2,0 g/100 g ; poivrons 1,2 g/100 g ou tomates 1,2 g/100 g.

13. Procédé mis en œuvre par ordinateur selon la revendication 10, dans lequel les denrées alimentaires riches en fibres comprenant des légumineuses contenant des fibres dans une quantité d'au moins environ 5,0 g à 9,0 g pour 100 g sont choisies dans le groupe comprenant : haricots noirs 8,7 g/100 g ; pois cassés 8,3 g/100 g ; lentilles 7,9 g/100 g ; pois chiches 7,6 g/100 g ; haricots rouges 6,4 g/100 g ; haricots cuits 5,5 g/100 g ; haricots de Lima 5,3 g/100 g ; ou fèves edamames 5,2 g/100 g.

14. Procédé mis en œuvre par ordinateur selon la revendication 10, dans lequel les denrées alimentaires riches en fibres comprenant des céréales complètes contenant des fibres dans une quantité d'au moins environ 2,8 g à 14,5 g par 100 g sont choisies dans le groupe comprenant : orge 17,0 g/100 g ; farine de céréales complètes 11 g/100 g ; avoine 10,6 g/100 g ; quinoa 2,8 g/100 g ; ou maïs soufflé 14,5 g/100 g.

15. Procédé mis en œuvre par ordinateur selon la revendication 10, dans lequel les denrées alimentaires riches en fibres comprenant des fruits à coque et des graines contenant des fibres dans une quantité d'au moins environ 7,0 g à 35,5 g par 100 g sont choisies dans le groupe comprenant : graines de chia 34,4 g/100 g ; graines de citrouille 18,4 g/100 g ; amandes 12,5 g/100 g ; pistaches 10,0 g/100 g ; noix de coco 9,0 g/100 g ; graines de tournesol 8,6 g/100 g ; ou noix 7,0 g/100 g.

16. Procédé mis en œuvre par ordinateur selon la revendication 10, dans lequel les denrées alimentaires riches en fibres comprenant des pommes de terre avec la peau contenant des fibres dans une quantité d'au moins 2,5 g par 100 g.

17. Procédé mis en œuvre par ordinateur selon la revendication 10, dans lequel les denrées alimentaires riches en fibres comprenant du chocolat noir contenant au moins 70 % de cacao contenant des fibres dans une quantité d'au moins environ 10,9 g par 100 g.

18. Procédé mis en œuvre par ordinateur selon la revendication 9, dans lequel la recommandation de fibres personnalisée est sous forme de fibres alimentaires consommables et d'un complément en fibres.

19. Procédé mis en œuvre par ordinateur selon la revendication 18, dans lequel la recommandation de fibres personnalisée d'un complément en fibres est choisie parmi un supplément contenant des fibres de guar, du psyllium, du glucomannane ou des β-glucanes.

20. Procédé mis en œuvre par ordinateur selon l'une quelconque des revendications 1 à 19, comprenant :
(iii) la fourniture de la composition de fibres personnalisée.

21. Système pour la fourniture d'une recommandation de fibres personnalisée à un individu, comprenant :
(i) un module d'entrée pour déterminer un profil d'enzymes actives sur les glucides, CAZyme, dudit individu, les CAZymes étant des enzymes qui assemblent ou décomposent des oligosaccharides et des polysaccharides ;
(ii) un module de calcul pour calculer la relation entre le profil CAZyme dudit individu et un apport en fibres ; et
(iii) un module de recommandation pour recommander une composition de fibres personnalisée pour ledit individu, dans lequel
le système est configuré pour
utiliser un outil d'évaluation d'alimentation pour évaluer un apport alimentaire dudit individu, l'outil d'évaluation d'alimentation comprenant un questionnaire concernant des antécédents alimentaires, un instrument d'évaluation d'alimentation court, un questionnaire concernant une fréquence alimentaire ou des outils technologiques utilisés dans une évaluation d'apport alimentaire,
affecter ledit individu à un groupe de profils CAZyme de différents groupes de profils CAZyme à l'aide de l'apport alimentaire évalué dudit individu, les différents groupes de profils CAZyme étant des échantillons regroupés en fonction d'une similarité de leurs profils CAZymes et les différents groupes de profils CAZyme étant prédéfinis à l'aide d'échantillons fécaux et d'habitudes alimentaires de volontaires, et
concevoir la composition de fibres personnalisée et recommandée en analysant des CAZymes enrichis dans le groupe de profils CAZyme auquel ledit individu est affecté.

22. Système selon la revendication 21, comprenant en outre :
(iv) un module de sortie pour fournir la composition de fibres personnalisée.

23. Système selon la revendication 21 ou 22, dans lequel la composition de fibres personnalisée est choisie dans le groupe constitué de : produits alimentaires, produits à boire ou compléments alimentaires ou combinaison de ceux-ci dans un kit de pièces fourni à l'individu.
